# EUROPEAN PATENT APPLICATION

(11) **EP 3 974 029 A1**
(43) Date of publication of application: **30.03.2022**
(21) Application number: 20382850.4
(22) Date of filing: 25.09.2020
(51) Int. Cl.: A61P 31/14, A61K 35/17, C12N 5/0783

(54) **MEMORY T CELLS AS ADOPTIVE CELL THERAPY FOR VIRAL INFECTIONS**

(71) Applicant: Universidad Autónoma de Madrid, 28049 Madrid (ES); Fundación para la Investigación Biomédica del Hospital Universitario de la Paz (FIBHULP), 28046 Madrid (ES); Universidad Miguel Hernández de Elche, 03202 Elche, Alicante (ES); Fundación de la Comunitat Valenciana para la Gestión del Instituto de Investigación Sanitaria y Biomédica de Alicante (Isabial), 03010 Alicante (ES)
(72) Inventor: PÉREZ MARTÍNEZ, Antonio, 28029 Madrid (ES); SORIA, Bernat, 03202 Elche (Alicante) (ES); FERRERAS PUENTE, Cristina, 28029 Madrid (ES)
(74) Representative: Hoffmann Eitle

(57) **Abstract**

The present invention refers to a cell suspension comprising at least 90% of CD45RA- memory T cells, characterized in that the memory T cells are derived from blood of convalescent patients recovered from an infection with Coronavirus and have specific lymphocyte antiviral reactivity against Coronavirus antigens, for use in the treatment of immunocompromised patients suffering from lymphopenia.

## Description

### FIELD OF THE INVENTION

The present invention refers to the medical field. Particularly, it refers to a cell suspension comprising at least 90% of CD45RA- memory T cells, characterized in that the memory T cells are derived from blood of convalescent patients recovered from an infection with Coronavirus and have specific lymphocyte antiviral reactivity against Coronavirus antigens, for use in the treatment of immunocompromised patients suffering from lymphopenia.

### STATE OF THE ART

In August 2020 the World Health Organization has declared that there are over twenty-two million presumed or confirmed cases of COVID-19 worldwide, with a total of over 792.000 deaths worldwide in 216 countries.

To date, more than 15 million patients has recovered from COVID-19 with no specific treatment. The role of immune system in COVID-19 is crucial to understand the disease outcome. Antibodies generated after an infection can protect against other infections, but some studies are showing that for other coronaviruses, such as SARS-CoV-1 SARS specific antibodies became undetectable after 6 years. In fact, humoral responses in COVID-19 disease are often of limited durability, as seen with other human coronavirus epidemics.

So far, the only treatment for COVID-19 is supportive. The antiviral, remdesivir, has been recently approved to treat COVID-19 with a light improvement versus placebo in shortening the time to recovery in adults. Vaccine development actively pursues to develop active immunity through vaccine immunization, but there is uncertainty about their long-term and full protective immunity against SARS-CoV-2.

The short efficacy window and long side effects of antiviral drugs, which is more important in this vulnerable population drove us to look for new antiviral strategies.

So, in summary, there is an unmet medical need of finding effective treatments for the treatment of viral infections, particularly for the treatment of COVID-19, which, at the same time do not show relevant side effects for the patients.

Thus, the present invention is aimed at solving this problem by using memory T cells as adoptive cell therapy for the treatment of said viral infections, particularly COVID-19.

### DESCRIPTION OF THE INVENTION

### Brief description of the invention

The inventors of the present invention have identified and characterized a cell population of T-lymphocyte memory cells which specifically attack the SARS-CoV-2 virus but can be also useful for other viral diseases and may i) discharge patients from the hospital in few days or ii) used as prophylactic measure to protect vulnerable populations. Infusion of these cells is feasible, remains safe because low alloreactivity capacity, when at least one HLA marker coincides with the host, keeping a functional T cell population which provides passive immunity against pathogens, which could be an effective living antiviral drug against SARS-COV-2. Memory T-lymphocytes specific for SARS-CoV-2 may also be cryopreserved and thawed keeping its properties and defining a pharmaceutical "off-the-self' product available when needed.

Particularly, the present invention refers to a cell suspension comprising at least 90% of CD45RA- memory T cells, characterized in that the memory T cells are derived from blood of convalescent patients recovered from an infection with Coronavirus and have specific lymphocyte antiviral reactivity against Coronavirus antigens, for use in the treatment of immunocompromised patients suffering from lymphopenia. Infusion of these cells is feasible, remains safe because low alloreactivity capacity while keeping a functional T cell population which provides passive immunity against pathogens, which could be an effective living antiviral drug against COVID-19, particularly against SARS-COV-2 infections.

Particularly, the inventors of the present invention have identified the presence of a specific SARS-Cov-2 T cell population within the CD45RA- T memory cells which can be efficiently used for the treatment of immunocompromised patients suffering from lymphopenia.

So, in the context of the present invention, it is proposed to isolate this specific cell population from convalescent donors who have recovered from COVID-19, for instance by CD45RA depletion, and use it to treat hospitalized suffering from lymphopenia, preferably COVID-19 patients. It is important to note that, these cells will not only be able to fight against future viral infections but also contain a pool of cells with memory against other pathogens which were present in the convalescent donors. Consequently, since secondary infections have been detected in hospitalized COVID-19 patients, the infusion of this cell population will increase the pool of T cells with the ability to eliminate said other pathogens which were present in convalescent donors, while increasing the percentage of T cells in the lymphopenic patients and SARS-Cov-2 specific T cells.

So, in summary, the inventors of the present invention carried out the:
- Identification of a SARS-Cov-2 specific T cell population in CD45RA- memory T cells from COVID-19 convalescents.
- Isolation of the T cell population using a CD45RA depletion device. These memory T cells will not only be able to fight against SARS-CoV-2 but also against other pathogens because CD45RA- memory T contain in the donor T cell memory repertoire for other pathogens, which is an important comorbidity COVID-19 associated.
- Development of an "off the shelf' stockage, pharmacy biobank of CD45RA- memory T cells from COVID-19 convalescents with a wide and different MHC antigens in order to reach most of the diversity population.
- Introduction of adoptive passive immunotherapy intravenous infusion of CD45RA-memory T cells from COVID-19 convalescents, in donor-recipient pairs sharing at least 1 class I or class II MHC antigen.
- Development of optimal administration routes and protocols for passive immunotherapy with CD45RA- memory T cells from COVID-19 convalescents, as prophylaxis in vulnerable patients from COVID-19, or pre-emptive treatment in asymptomatic reservoirs which the aim of reduce the contagious period.

So, the first embodiment of the present invention refers to a cell suspension (hereinafter cell suspension of the invention) comprising at least 90% of CD45RA- memory T cells, characterized in that the memory T cells are derived from blood of convalescent patients recovered from an infection with Coronavirus and have specific lymphocyte antiviral reactivity against Coronavirus antigens, for use in the treatment of immunocompromised patients suffering from lymphopenia.

In a preferred embodiment, the memory T cells of the invention are derived from blood of convalescent patients recovered from an infection with SARS-CoV-2 and have specific lymphocyte antiviral reactivity against SARS-CoV-2 antigens. It is important to note that the cell suspension of the invention has shown immunogenicity for all the SARS-CoV-2 antigens (M, N y S), both independently or jointly.

In a preferred embodiment, at least 75% of the CD45RA- cells are CD3+ cells, at least 70% of the CD45RA- CD3+ cells are CD4+ and at least 10% of the CD45RA- CD3+ cells are CD8+. In a preferred embodiment, 75%-85% of the CD45RA- cells are CD3+ cells, 70%-85% of the CD45RA- CD3+ cells are CD4+ and 10%-30% of the CD45RA- CD3+ cells are CD8+. In a preferred embodiment, around 80% of the CD45RA- cells are CD3+ cells, around 83% of the CD45RA- CD3+ cells are CD4+ and around 14% of the CD45RA- CD3+ cells are CD8+.

In a preferred embodiment, at least 60% of the CD45RA- CD4+ cells are CD27+, at least 5% of the CD45RA- CD4+ cells are CD27- and at least 5% of the CD45RA- CD4+ cells are CD127lowCD25+. In a preferred embodiment, 60-90% of the CD45RA- CD4+ cells are CD27+, 5%-25% of the CD45RA- CD4+ cells are CD27- and 5%-25% of the CD45RA- CD4+ cells are CD127lowCD25+. In a preferred embodiment, around 89% of the CD45RA- CD4+ cells are CD27+, around 10% of the CD45RA- CD4+ cells are CD27- and around 11% of the CD45RA-CD4+ cells are CD127lowCD25+.

In a preferred embodiment, at least 50% of the CD45RA- CD8+ cells are CD27+ and at least 10% of the CD45RA- CD8+ cells are CD27-. In a preferred embodiment, 50-70% of the CD45RA- CD8+ cells are CD27+ and 10-40% of the CD45RA- CD8+ cells are CD27-. In a preferred embodiment, around 61% of the CD45RA- CD8+ cells are CD27+ and around 38% of the CD45RA- CD8+ cells are CD27-.

In a preferred embodiment, at least 10% of the CD3+ cells are HLADR+, at least 0.5% of the CD3+ cells are CD69^{high+} and at least 10% of the CD3+ cells are CD25+. In a preferred embodiment, 10-30% of the CD3+ cells are HLADR+, at 0.5-3% of the CD3+ cells are CD69^{high+} and 10-80% of the CD3+ cells are CD25+. In a preferred embodiment, around 19% of the CD3+ cells are HLADR+, around 0.5% of the CD3+ cells are CD69^{high+} and around 60% of the CD3+ cells are CD25+.

In a preferred embodiment, at least 5% of the CD4+ cells are HLADR+, at least 0.2% of the CD4+ cells are CD69+, and at least 20% of the CD4+ cells are CD25+. In a preferred embodiment, 5-20% of the CD4+ cells are HLADR+, 0.2-1% of the CD4+ cells are CD69+, and 20-80% of the CD4+ cells are CD25+. In a preferred embodiment, around 16% of the CD4+ cells are HLADR+, around 0.4% of the CD4+ cells are CD69+, and around 66% of the CD4+ cells are CD25+.

In a preferred embodiment, at least 5% of the CD8+ cells are HLADR+, at least 0.15% of the CD8^{high+} cells are CD69+, and at least 4% of the CD8+ cells are CD25+. In a preferred embodiment, 5-40% of the CD8+ cells are HLADR+, 0.15-0.9% of the CD8^{high+} cells are CD69+, and 4-12% of the CD8+ cells are CD25+. In a preferred embodiment, around 29% of the CD8+ cells are HLADR+, around 0.3% of the CD8^{high+} cells are CD69+, and around 9% of the CD8+ cells are CD25+.

In a preferred embodiment, less than 5% of the CD3+ cells are NKG2A+, less than 1% of the CD3+ cells are PD1+, less than 0.1% of the CD4+ cells are NKG2A+, less than 5% of the CD4+ cells are PD1+, less than 20% of the CD8+ cells are NKG2A+ and less than 5% of the CD8+ cells are PD1+. In a preferred embodiment, 2-5% of the CD3+ cells are NKG2A+, 0.5-1% of the CD3+ cells are PD1+, 0-0.1% of the CD4+ cells are NKG2A+, 1-5% of the CD4+ cells are PD1+, 5-20% of the CD8+ cells are NKG2A+ and 0-5% of the CD8+ cells are PD1+. In a preferred embodiment, around 1% of the CD3+ cells are NKG2A+, around 0.8% of the CD3+ cells are PD1+, around 0.1% of the CD4+ cells are NKG2A+, around 1.4% of the CD4+ cells are PD1+, around 10% of the CD8+ cells are NKG2A+ and around 0% of the CD8+ cells are PD1+.

In a preferred embodiment, at least 60% of the CD3+ cells are CCR7+, at least 1% of the CD3+ cells are CD103+, at least 50% of the CD4+ cells are CCR7+, at least 0.5% of the CD4+ cells are CD103+, at least 30% of the CD8+ cells are CCR7+ and at least 2% of the CD8+ cells are CD103+. In a preferred embodiment, 60-90% of the CD3+ cells are CCR7+, 1-5% of the CD3+ cells are CD103+, 50-90% of the CD4+ cells are CCR7+, 0.5-5% of the CD4+ cells are CD103+, 30-60% of the CD8+ cells are CCR7+ and 2-10% of the CD8+ cells are CD103+. In a preferred embodiment, around 81% of the CD3+ cells are CCR7+, around 2% of the CD3+ cells are CD103+, around 88% of the CD4+ cells are CCR7+, around 1% of the CD4+ cells are CD103+, around 45% of the CD8+ cells are CCR7+ and around 6% of the CD8+ cells are CD103+.

In a preferred embodiment, the expression of the activation markers CD69, CD25, HLADR and/or CD103 is characterized by a fold change of at least 1.5, preferable at least 2, when compared with the expression measured in the basal cell population and consequently show an improved expression of activation and migration markers to the respiratory track. Particularly, the cells of the invention have been stimulated overnight (o.n-72h) with 10-50 ng/ml interleucina-15 (IL-15). IL-15 facilitates the homeostasis of T cells and promotes migration of effector CD8+ T cells to the respiratory truck and a functional and protective CD4 T cells. An increase in activation markers (CD69, CD25, HLADR and/or CD103) is overserved in the present invention and a light increase of T cell exhaustion marker (NKG2A and PD1). This activation increases the expression of Integrin, alpha E (ITGAE), like T cell resident memory which allow to virus clearance.

In particularly preferred embodiment, the phenotype of the cell population of the invention is illustrated in **Table 3** below.

In a preferred embodiment, the cell suspension of the invention is used as an adoptive third-party off-the-shelf treatment in patients suffering from lymphopenia caused by a viral infection, preferably caused by Coronavirus.

In a preferred embodiment, the cell suspension of the invention is used in the treatment of immunocompromised patients suffering from lymphopenia caused by a viral infection, preferably caused by SARS-CoV-2.

The cells of the invention may be infused fresh or cryopreserved. Once thawed, they can be intravenously infused as standard donor lymphocyte infusion (DLI). Furthermore, as explained above, the cells of the invention can be IL-15 cytokine activated to increase activation phenotype and favouring upper and lower respiratory tract homing. In addition, both products, stimulated and unstimulated, can also administered locally in order to reach and colonize respiratory tract where the COVID-19 entry door. With this treatment we may protect from SARS-CoV-2 infection vulnerable population as elderly and decrease the time of positive PCR in asymptomatic SARS-CoV-2 positive population. With an increasing number of CD45RA-memory pharmacy biobank from COVID-19 convalescents containing different MHC class I and II typing, the probability that a recipient in the same ethnic group would share at least one donor HLA allele or one haplotype could be higher.

In a preferred embodiment, the cell suspension of the invention is administered either by nebulization or locally applied. In a preferred aspect, the cell suspension of the invention which are characterized by an improved expression of the activation markers CD69, CD25, HLADR and/or CD103 (fold change of at least 1.5), is administered locally in the oral, nasal or ocular mucosae since it shows improved migration capacity to the respiratory track. Thus, the cells can be nebulized using a jet or air compression system to localize the cells in the upper respiratory tract which is the main entry of SARS-CoV-2 or locally instilled in the oral, nasal or ocular mucosae. It is herein proposed to treat vulnerable COVID-19 negative population as elderly as prophylaxis, SARS-CoV-2 asymptomatic patients in order to decrease the infective period and the risk of COVID-19 development. Rationale for this approach is because most common airborne pathogens as SARS-CoV-2, in the human primarily infect the upper respiratory tract (URT). The nasal and tonsil associated lymphoid tissues is a mucosal inductive site for humoral and cellular immune responses who is plenty by memory CD4+ T cells, suggesting that it is optimized to initiate memory recall responses, rather than initiate primary T cell responses. CD45RA- T cell memory containing SARS-CoV-2 specific T memory cells could be a potent antiviral agent and is compatible with vibrating air compression nebulization delivery.

In a particular embodiment of this invention, the adoptive immunotherapy is applied in combination with natural killer cells (NK cells) in order to reconstruct the immune system of vulnerable patients.

Particularly, the infusion of the minimally manipulated CD45RA- T cell memory of the invention does not require GMP facilities, making this approach a widely available blood bank procedure. Moreover, this procedure would be cost-effective considering the actual pandemic and the high cost and minimal beneficial of antiviral drugs. The cells of the invention can be easily obtained and processed from COVID-19 convalescent donors and they will be immediately available off-the-shelf to treat and prevent severe cases of COVID-19 patients.

In a preferred embodiment of this invention this adoptive antiviral immunotherapy is applied in combination with mesenchymal stromal cells in order to prevent the cytokine storm and promote tissue regeneration of a patient.

In a preferred embodiment, the cell suspension of the invention is pre-treated with interleukins or factors of the following list: Akt inhibitors, lithium chloride, calcium channel agonists and other factors that promote proliferation, maturation and activation of T-memory lymphocytes. So, the cell suspension of the invention may be used as an Advanced Therapy Medicinal Product, wherein the cell suspension is preferably pre-treated with interleukin-15 at a concentration between 10 ng/ml and 100 ng/ml, but preferably 50ng/ml.

In a preferred embodiment, the cell suspension of the invention is cultured and expanded in a xeno-free and human component free culture media.

In a preferred embodiment, of the cell suspension of the invention is thawed in the presence of an inhibitor of the sodium-proton exchange such as amiloride.

In a preferred embodiment, the cell suspension of the invention is administered in combination with natural killer cells or with mesenchymal stromal cells.

The second embodiment of the present invention refers to a pharmaceutical composition comprising the cell suspension of the invention and, optionally, pharmaceutically acceptable excipients and/or carriers.

The last embodiment of the invention refers to a method for treating immunocompromised patients suffering from lymphopenia which comprises the administration of a therapeutically effective amount of the cell suspension of the invention.

For the purpose of the interpretation of the present invention the following terms are defined:
- The term "lymphopenia" refers to a medical condition when the number of lymphocytes per volume of blood (generally expressed per microliter) are reduced as compared with a threshold number taken from healthy individuals. This haematological parameter depends on the age of a patient and the test method used. Normal ranges provided for different age groups can differ depending on the laboratory and test technology. Nevertheless, the generally used normal ranges for adults are approximately 20-40% and 1,500-4,500 lymphocytes/µL *[*Naeim F, Rao PN, Song SX, Grody WW. Atlas of Hematopathology. Morphology, Immunophenotype, Cytogenetics, and Molecular Approaches. 1st edition. San Diego, CA: Elsevier Science Publishing Co Inc.; 2013. pp. 627-628][Longo DL, Fauci A, Kasper D, Hauser S, Jameson JL, Loscalzo J.Harrison's Principles of Internal Medicine. 18th edition. Columbus, OH: McGraw-Hill; 2011].
- The term "treatment" refers to the medical care given to a patient. It can be, according to the present invention, a prophylactic or pre-emptive treatment for the prevention of the viral infection in vulnerable patients, or an active treatment for patients suffering from a viral infection.
- The term "adoptive third-party off-the-shelf treatment" refers to transfer HLA partially matched memory T cells from a COVID-19 convalescents.
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included with the cell suspension of the invention and that causes no significant adverse toxicological effects to the patient.
- By "therapeutically effective dose or amount" of a composition comprising the cell suspension of the invention is intended an amount that, when administered as described herein, brings about a positive therapeutic response in a subject having a viral infection. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

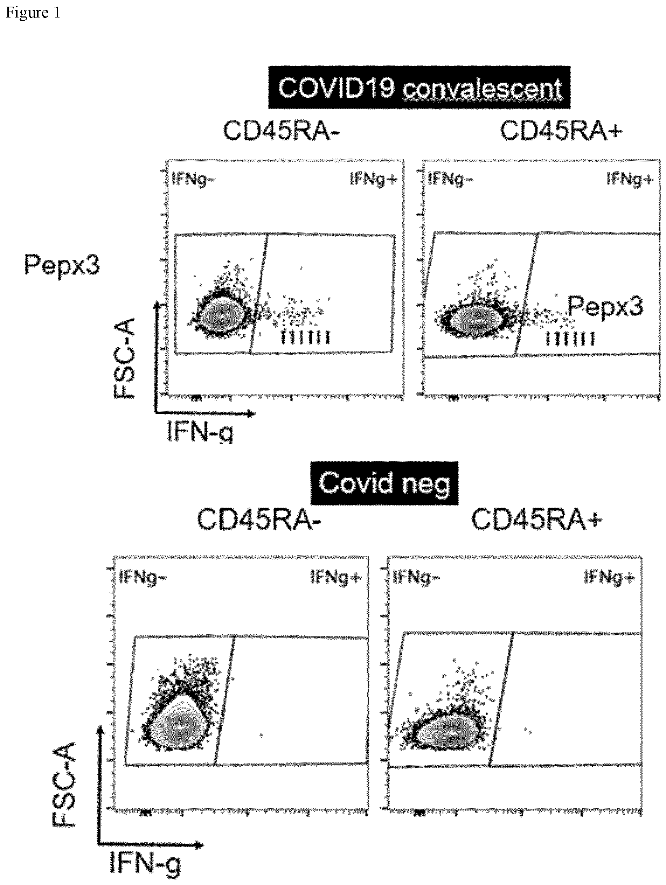

**Figure 1**. Expression of IFN-γ positive T cells in CD45RA- memory T cells vs CD45RA+ T cells in COVID-19 convalescents vs COVID-19 negative control. Peripheral blood mononuclear cells from COVID-19 convalescents (above) and COVID-19 negative control (below), were incubated with specific SARS-CoV-2 peptides, membrane peptide (M), nucleocapside antigen (N) and spike antigen (S). It can be observed (black arrows) how COVID-19 convalescents express IFN-γ positive T cells (both in CD45 RA- memory and CD45RA+ naive T cells). However, COVID-19 negative control does not express IFN-g positive T cells either CD45RA-memory neither CD4RA+ naive T cells.
**Figure 2****.** Expression of IFN-γ positive T cells in CD4, CD8 subsets in both CD45RA- memory T cells (above) vs CD45RA+ naive T cells (below) in COVID-19 convalescents. Both T cell subset population contained IFN-γ positive T cells, being significantly higher in T cell CD45RA-memory CD4+ T cells.

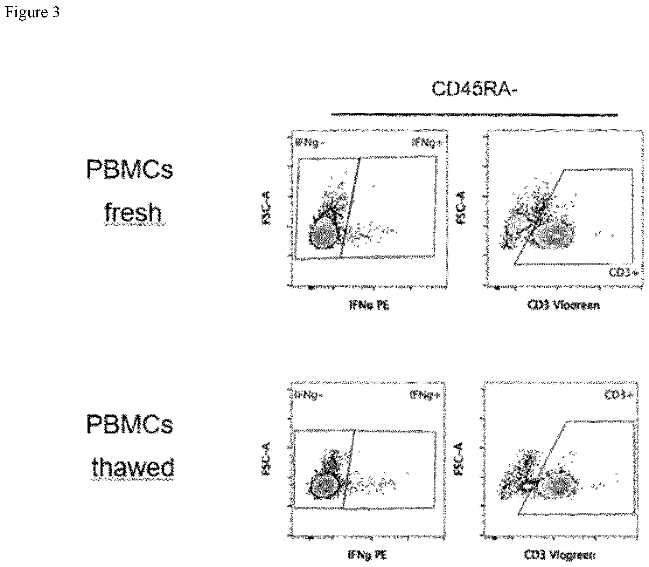

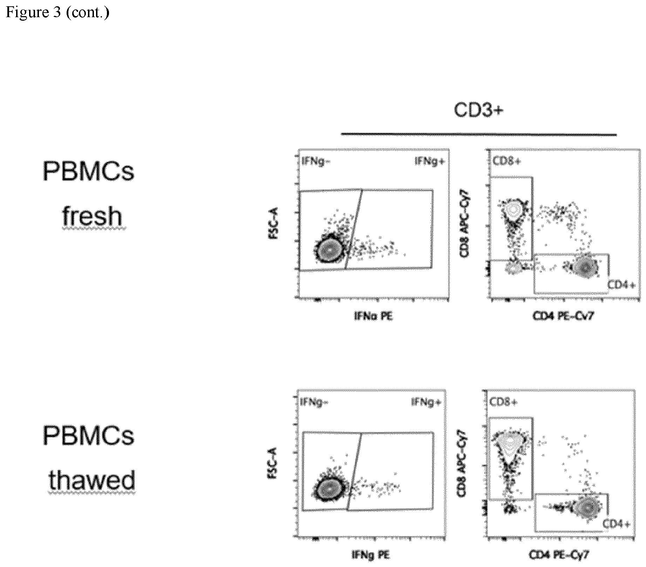

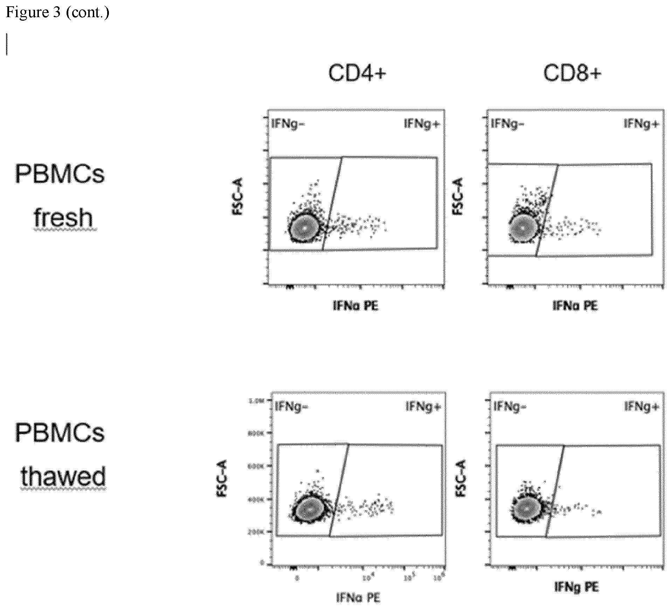

**Figure 3****.** Expression of IFN-γ positive T cells in CD45RA- memory T cells, CD3+ and CD4+, CD8+ subsets in fresh and thawed PBMCs from a COVID-19 convalescent. Cryopreservation do not affect SARS-CoV-2 specific release of IFN-γ.
**Figure 4****.** Expression of CD27 positive central memory phenotype (CM), vs CD27 negative (EM) effector memory (EM) phenotype in both CD4 and CD8 T cell subsets from CD45RA-memory T cells in COVID-19 convalescents. The ratio CM/EM is higher in CD4 T cell subset than in CD8.
**Figure 5**. Expression of HLADR activation marker resting and after o/n and 3 days IL-15 activation. The fold increase after incubation was 1.2 o.n and 2.6 after 3 days of IL-15 stimulation.
**Figure 6****.** Expression of CD69 activation marker resting and after o/n and 3 days IL-15 activation. The fold increase after incubation was 3.2 o.n and 29.6 after 3 days of IL-15 stimulation, which apart from an early activation marker is consider improving T cell migration to respiratory track.
**Figure 7****.** Expression of CD25 activation marker resting and after o/n and 3 days IL-15 activation. The fold increase after incubation was 1.5 o.n and 8.6 after 3 days of stimulation. CD4CD127lowCD25 T reg is around 7%, increasing lightly after three days of IL-15 stimulation
**Figure 8****.** Expression of NKG2A exhaustion marker resting and after o/n and 3 days of IL-15 activation. The fold increase after incubation was increased after three days of IL-15 stimulation to 1.4.
**Figure 9****.** Expression of PD-1 exhaustion marker resting and after o/n and 3 days of IL-15 activation. The fold increase after incubation was only increased until three days of IL.15 stimulation to 8.7.
**Figure 10****.** Expression of CCR7 chemokine in resting and after o/n and 3 days of IL-15 activation. No modification after IL-15 stimulation, being consistent with CD27 expression
**Figure 11****.** Expression of CD103 integrin in resting and after o/n and 3 days of IL-15 activation. The fold increase after incubation was only increased until three days of IL-15 stimulation to 1.2.
**Figure 12****.** COVID-19 Tmem lymphocyte pharmacy- biobank a CD45RA- memory T cells containing SARSCOV-2 specific T cells (Lymphoteca). At least 30 aliquots may be obtained from 0.1.1x10⁶/kg.
**Figure 13****.** Scores showing disease severity (National Early Warning Score NEWS and 7-point ordinal scale) over time for the patients enrolled in this study.
**Figure 14****.** Lymphocyte and IL-6 values over time for Patient #1
**Figure 15****.** Lymphocyte and IL-6 values over time for Patient #2

### Detailed description of the invention

The present invention is illustrated by means of the examples below without the intention of limiting its scope of protection.

### Example 1. Results.

### Example 1. Material and Methods.

### Example 1.1. Donors.

**Table 1** shows the patients' characteristics. Six convalescent donors and two healthy controls were included. Two patients presented with bilateral pneumonia needing one of them supportive treatment. None of them were hospitalized.

Eligibility criteria included age 21 to 65, a history of COVID-19 with documented positive reverse-transcriptase polymerase chain reaction (RT-PCR) test for SARS-CoV-2 that tested negative recently. Two healthy donors were enrolled who have not been exposed to COVID-19 patients, never had symptoms related to COVID-19 and have been tested negative for anti-SARS-CoV-2 antibodies.

All the participants gave written consent with approval by the hospital Institution Review Board.

**Table 1**

| **n** | **6** |
|---|---|
| Age (years), mean (range) | 37 (23-41) |
| Gender (female/male) | 3/3 |
| Days to SARS-Cov-2 negative OCR (range) | 13 (5-17) |
| Bilateral pneumonia, n(%) | 2 (33.3%) |
| Ambulatory, n(%) | 6 (100%) |

| **Treatment** | |
|---|---|
| Acetaminophen | 5 |
| Hydroxicloroquina+azytromicina+remdesivir | 1 |
| Hydroxicloroquina+azytromicina | 1 |

### Example 1.2. Cell processing for IFN-γ assay

PBMCs from healthy donors and patients were isolated by density gradient centrifugation using Ficoll-Paque (GE Healthcare, Illinois, Chicago, USA). Briefly, cells were rested overnight (o/n) at 37°C in TexMACS Medium (Miltenyi Biotec, Bergisch Gladbach, Germany) supplemented with 10% AB serum (Sigma-Aldrich, Saint Louis, Missouri, USA). Next day 1x10⁶ cells were stimulated with pooled or individual overlapping peptides of SARS-Cov-2 at a final concentration of 0.6 nmol/ml in the presence of plate bound stimulator OKT3 at a final concentration of 2.8 µg/ml (mouse anti human CD3 Clone OKT3 BD Biosciences) and co-stimulation with CD28/CD49d at a final concentration of 5 µg/ml (anti-human CD28/CD49d Purified Clone L293 L25 BD Biosciences. The peptide pools were short 15-mer peptides with 11 amino acid overlaps which can bind MHC class I and class II complexes and thus were able to stimulate both CD4+ and CD8+ T cells. The peptides cover the immunodominant sequence domains of the surface glycoprotein S, and the complete sequence of the nucleocapsid phosphoprotein N and the membrane glycoprotein M (GenBank MN908947.3, Protein QHD43416.1, Protein QHD43423.2, Protein QHD43419.1) (Miltenyi Biotec, Germany). After 5 hours stimulation, the cells were labelled with the IFN-γ Catch Reagent (IFN-γ Secretion Assay-Detection Kit, human Miltenyi Biotec) containing bispecific antibodies for CD45 and IFN-γ which was secreted by the stimulated target cells. After the secretion phase, the cell surface-bound IFN-γ was targeted using IFN-γ PE antibody.

Negative and positive controls were run in parallel.

The analysis of the cell composition in the T cells specifically activated by SARS-Cov-2 in the IFN- γ assay was performed IFN-γ Secretion Assay-Detection Kit, human Miltenyi Biotec and cytokine response was background subtracted.

### Example 1.3. Donor selection, and CD45RA+ T cell depletion.

The convalescent donor was selected based on the secretion of IFN-γ upon activation with the peptivator and the HLA typing.

Non-mobilised apheresis was obtained from the convalescent donor at the Bone Marrow Transplant and Cell Therapy Unit for University Hospital La Paz by using CliniMACS Plus device (Milteny Biotec). The donor gave written informed consent in accordance with the Declaration of Helsinki protocol, and the study was performed according to the guidelines of the local ethics committee. The donor complies with the requirements regarding quality and safety for donation, obtaining, storage, distribution, and preservation of human cells and tissues under the Spanish specific regulation. CD45RA+ cells were depleted by immunomagnetic separation using CliniMACS CD45RA Reagent and CliniMACS Plus system, both from Miltenyi Biotec, following manufacturer instructions. CD45RA- cells were frozen using autologous plasma and stored. The viability and purity of CD45RA- fraction were analysed by flow cytometry (FCM).

### Example 1.4. Cell processing to obtain an activated phenotype of memory T cells

CD45RA- memory T cells from the convalescent donor where thawed and incubated in Tex MACs media TexMACS Medium (Miltenyi Biotec, Germany) supplemented with 5% AB serum (Sigma-Aldrich, Saint Louis, Missouri, USA) plus 50 ng/ml of IL15 o/n and for 72 hours. After that time the cells were harvested and the phenotypic assay was performed. The same culture without IL15 was run in parallel as a control.

### Example 1.5. Flow cytometry.

Cell surface staining was performed for 20 minutes at 4 degrees with the following fluorochrome conjugated antibodies titrated to their optimal concentrations: 7AAD (BD Horizon), CD45RA FITC (BD Pharmingen), CD3 viogreen (Miltenyi Biotec), CD4 PECy7 (BD Pharmingen), CD8 APC Cy7 (BD Pharmingen), CD27 APC (BD Pharmingen). For the Treg panel CD25 BV421 (BD Horizon), and CD127 PE-CF594 (BD Horizon), was used. For the activation panel HLA-DR BV 421 (BD Pharmingen), CD69 BV421 (Biolegend), CD25 BV421 (BD Horizon), was used. For the exhaustion panel PD1 AF700 (Biolegend), NKG2D BV421 (Biolegend) was used. For the cytokine panel CD103 BV421 (BD Horizon) and CCR7 PE-CF594 (BD Horizon) was used. An average of 200.000 cells was acquired. Cell acquisition was performed using a FACS Canto. The analysis was performed using FlowJo 9.9.6 (FlowJo LLC).

### Example 2. Results.

### Example 2.1. Characteristics of the convalescent donors

Six convalescent donors who recovered from COVID-19 and two unexposed healthy controls were recruited. **(Table 1).** Median age 37 years old (range 23-41), three were females and three were males. SARS-CoV-2 PCR test was performed for all of them in nasopharyngeal samples. Two of them presented with bilateral pneumonia but no hospitalisation was needed. Just one of them needed Hydroxicloroquina + azytromicina + remdisivir and another one needed hydroxicloroquina and azytromicina **(Table 1).**

### Example 2.2. Characterisation of the CD45RA- subpopulation

To study the existence of T cells specific for SARS-CoV-2 we looked at the IFN-γ secreted by T cells after exposure to the overlapping peptides of SARS-CoV-2 in both subsets naive CD45RA+ and memory CD45RA- T cells in PBMCs. The median days that passed after they tested negative for SARS-CoV-2 was 21 days **(Table 1).** We observed that after a short exposure of 5 hours to the SARS-CoV-2 peptides, they all showed reactivity for the single peptides M, N and S and the pool of the three of them when compared to the healthy individuals **(Table 2** and **Figure 1****).** It is important to highlight that there is not a synergistic effect on the percentage of IFN-γ when the three peptides are mixed when compared to the single peptides **(Table 2).** The media CD45RA-CD3+ population in the convalescent donors was 90%. The memory CD45RA-CD3+ population expressed a higher proportion of IFN-γ when compared to the naive CD45RA+CD3+ population (1.12% vs 0.4%) **(Table 2)** when co-culture with the three peptides. This result shows the existence of a functional population of memory T cells against SARS-CoV-2. The healthy donors didn't express IFN-γ in any of the conditions studied **(Table 2).**

**Table 2**

| | | **C1** | **C2** | **MEDIA Pepx3** | **SD** | **D1** | **D2** | **D3** | **D4** | **D5** | **D6** | **MEDIA Pepx3** | **SD** |
|---|---|---|---|---|---|---|---|---|---|---|---|---|---|
| **CD45RA-** | % CD45RA- | 37.7 | 22.2 | 29.93 | 11.0 | 31.2 | 24.1 | 18.2 | 43.9 | 19.9 | 33.2 | 28.41 | 9.7 |
| | IFNγ+ | 0.00 | 0.00 | 0.00 | 0.00 | 0.77 | 0.99 | 3.00 | 0.30 | 0.90 | 0.87 | 1.14 | 0.9 |
| CD45RA-CD3+ | % CD3+ | 61.7 | 78.4 | 70.04 | 11.8 | 93.8 | 93.5 | 94.5 | 77.1 | 86.6 | 94.5 | 90.01 | 7.0 |
| | IFNγ+ | 0.00 | 0.00 | 0.00 | 0.00 | 0.69 | 0.79 | 3.03 | 0.34 | 1.01 | 0.83 | 1.12 | 1.0 |
| CD45RA-CD8+ | % CD8+ | 17.1 | 10.0 | 13.56 | 5.1 | 17.0 | 22.2 | 16.3 | 26.6 | 11.9 | 35.1 | 21.51 | 8.4 |
| | IFNγ+ | 0.00 | 0.00 | 0.00 | 0.00 | 0.38 | 0.46 | 3.45 | 0.12 | 0.70 | 0.00 | 0.85 | 1.3 |
| | % CD8+ CM | 87.9 | 87.3 | 87.58 | 0.4 | 86.5 | 65.7 | 76.3 | 88.0 | 90.0 | 42.5 | 74.84 | 18.3 |
| | IFNγ+ | 0.00 | 0.00 | 0.00 | 0.00 | 0.31 | 0.33 | 2.88 | 0.11 | 0.64 | 0.48 | 0.79 | 1.0 |
| | % CD8+ EM | 12.1 | 12.7 | 12.37 | 0.4 | 13.4 | 34.3 | 23.7 | 11.9 | 9.9 | 57.5 | 25.11 | 18.3 |
| | IFNγ+ | 0.00 | 0.00 | 0.00 | 0.00 | 0.00 | 0.79 | 5.58 | 0.00 | 0.00 | 0.00 | 1.06 | 2.2 |
| CD45RA-CD4+ | % CD4+ | 68.2 | 86.7 | 77.42 | 13.1 | 76.0 | 75.8 | 79.3 | 70.6 | 80.1 | 60.6 | 73.74 | 7.2 |
| | IFNγ+ | 0.00 | 0.00 | 0.00 | 0.00 | 0.81 | 0.91 | 2.93 | 0.43 | 1.11 | 1.32 | 1.25 | 0.9 |
| | % CD4+ CM | 89.1 | 81.5 | 85.30 | 5.4 | 70.8 | 86.1 | 90.3 | 82.9 | 91.9 | 90.8 | 85.46 | 8.0 |
| | IFNγ+ | 0.00 | 0.00 | 0.00 | 0.00 | 0.56 | 1.01 | 2.93 | 0.47 | 1.13 | 1.44 | 1.26 | 0.9 |
| | % CD4+ EM | 10.9 | 18.5 | 14.67 | 5.4 | 29.2 | 13.9 | 9.7 | 17.1 | 8.0 | 9.2 | 14.53 | 8.0 |
| | IFNγ+ | 0.00 | 0.00 | 0.00 | 0.00 | 1.37 | 0.39 | 3.17 | 0.21 | 1.35 | 0.98 | 1.25 | 1.1 |
| **CD45RA+** | % CD45RA+ | 61.9 | 77.8 | 69.87 | 11.3 | 68.7 | 75.9 | 81.9 | 56.0 | 80.3 | 66.8 | 71.57 | 9.7 |
| | IFNγ+ | 0.00 | 0.02 | 0,01 | 0.00 | 0.67 | 0.93 | 6.47 | 0.10 | 0.52 | 0.32 | 1.50 | 2.5 |
| CD45RA+ CD3+ | % CD3+ | 70.4 | 78.1 | 74.25 | 5.4 | 52.9 | 79.1 | 61.0 | 59.8 | 74.6 | 79.2 | 67.75 | 11.3 |
| | IFNγ+ | 0.02 | 0.01 | 0.02 | 0.00 | 0.40 | 0.27 | 1.25 | 0.06 | 0.23 | 0.17 | 0.40 | 0.4 |
| CD45RA+ CD8+ | % CD8+ | 22.8 | 24.4 | 23.56 | 1.1 | 56.5 | 27.2 | 49.9 | 32.1 | 33.1 | 38.6 | 39.57 | 11.3 |
| | IFNγ+ | 0.00 | 0.00 | 0.00 | 0.00 | 0.13 | 0.46 | 1.69 | 0.09 | 0.41 | 0.21 | 0.50 | 0.6 |
| | % CD8+ Naive | 72.8 | 83.6 | 78.21 | 7.6 | 76.6 | 84.6 | 80.4 | 95.9 | 80.8 | 44.8 | 77.19 | 17.2 |
| | IFNγ+ | 0.00 | 0.00 | 0.00 | 0.00 | 0.17 | 0.22 | 0.51 | 0.10 | 0.29 | 0.15 | 0.24 | 0.1 |
| | % CD8+ TEMRA | 27.1 | 16.4 | 21.79 | 7.6 | 23.4 | 15.4 | 19.6 | 4.1 | 19.2 | 55.2 | 22.81 | 17.2 |
| | IFNγ+ | 0.00 | 0.00 | 0.00 | 0.00 | 0.07 | 1.81 | 7.02 | 0.00 | 1,37 | 0,29 | 1.76 | 2.7 |
| CD45RA+ CD4+ | % CD4+ | 70.3 | 70.1 | 70.18 | 0.1 | 32.4 | 70.3 | 47.3 | 65.9 | 59,5 | 54,4 | 54.95 | 13.8 |
| | IFNγ+ | 0.00 | 0.00 | 0.00 | 0.00 | 0.47 | 0.14 | 0.55 | 0.06 | 0,22 | 0,09 | 0.26 | 0.2 |
| | % CD4+ Naive | 99.5 | 98.7 | 99.13 | 0.6 | 95.7 | 99.5 | 99.3 | 99.6 | 99,4 | 99,2 | 98.78 | 1.5 |
| | IFNγ+ | 0.00 | 0.00 | 0.00 | 0.00 | 0.21 | 0.13 | 0.52 | 0.07 | 0,21 | 0,09 | 0.21 | 0.2 |
| | % CD4+ TEMRA | 0.5 | 1.3 | 0.88 | 0.6 | 4.3 | 0.5 | 0.7 | 0.4 | 0,7 | 0,8 | 1.23 | 1.5 |
| IFNγ+ | | 0.00 | 0.00 | 0.00 | 0.00 | 5.77 | 0.00 | 2.30 | 0.00 | 0.00 | 0.00 | 1.35 | 2.4 |

| | | | | | | | | | | | | | |
|---|---|---|---|---|---|---|---|---|---|---|---|---|---|
| *Phenotypic characterization of the healthy controls and the convalescent donors after co-culture with the mixture of the three peptides (M, N, S)C*= *healthy controls. D*= *convalescent donors SD*= *Standard deviation Pepx3*= *mixture of the single peptides M,N,S.* | | | | | | | | | | | | | |

We didn't find a correlation between severity of symptoms and percentage of IFN- γ secreted.

### Example 2.3. Characterisation of the CD4+ CD8+ T cell subsets.

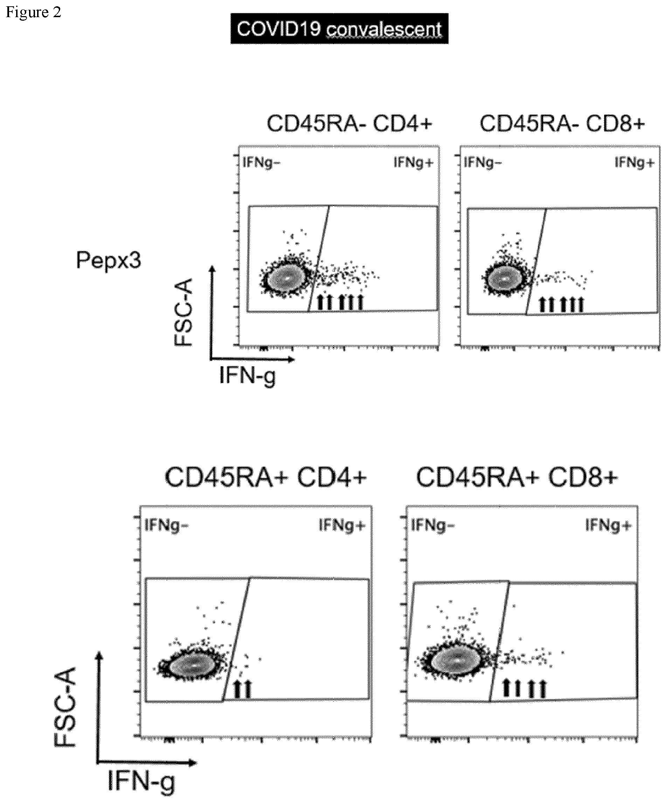

The percentage of CD4+ and CD8+ cells within the CD45RA-CD3+ population was +73.74% and 21.5 % respectively. Among all subsets we observed that CD45RA-CD4+ T cells expressed the highest proportion of IFN-y+ cells (1.25%) when compared to the CD45RA-CD8+ (0.85%), CD45RA+CD4+ (0.26%) and CD45RA+CD8+ (0.50%) populations regardless the exposure was to either the three peptides combined or each of them separately **(Table 2** and **Figure 2****).**

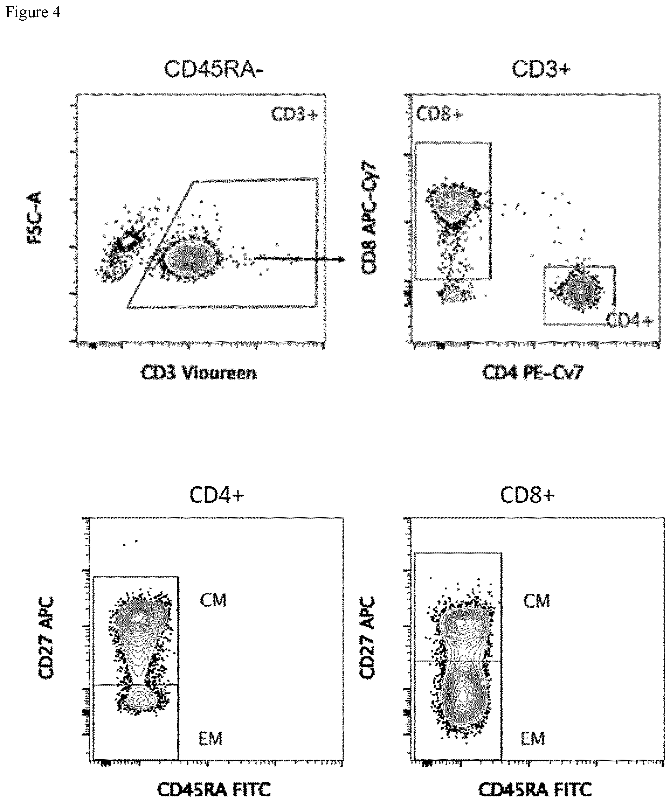

Within the T central memory (CM) (CD45RA-CD3+CD27+) and T effector memory (EM) (CD45RA-CD3+CD27-) compartments most of the CD45RA-CD3+CD4+ and CD45RA-CD3+CD8+ cells from convalescent donors were CM (85.4% and 74.8% respectively) as in the controls (85.3% and 87.6% respectively). The expression of IFN-γ was then studied within those population. We found a higher expression within the CD4+ (CM) (1.26%) and CD4+ EM (1.25%) when compared to the CD8+ CM (0.79%) CD8+ EM (1.06%) subpopulations **(Table 2 and** **Figure 4****).**

### Example 2.4. Phenotype of CD45RA- memory T cells from a convalescent donor.

Blood donor apheresis was performed followed by CD45RA+ T cell depletion. The CD45RA-fraction was cryopreserved and phenotypically characterised **(Table 3).**

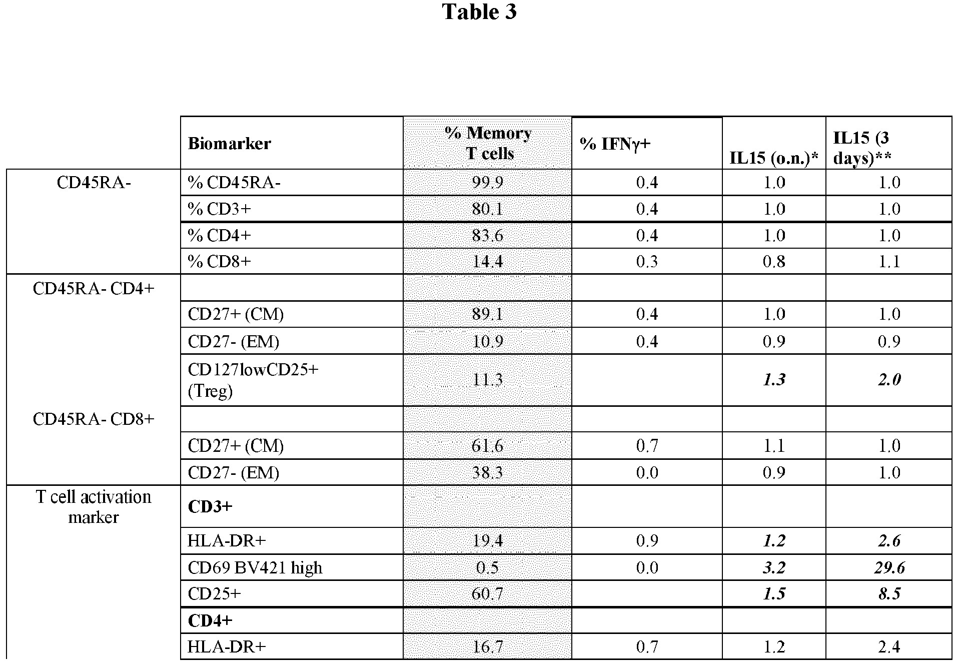

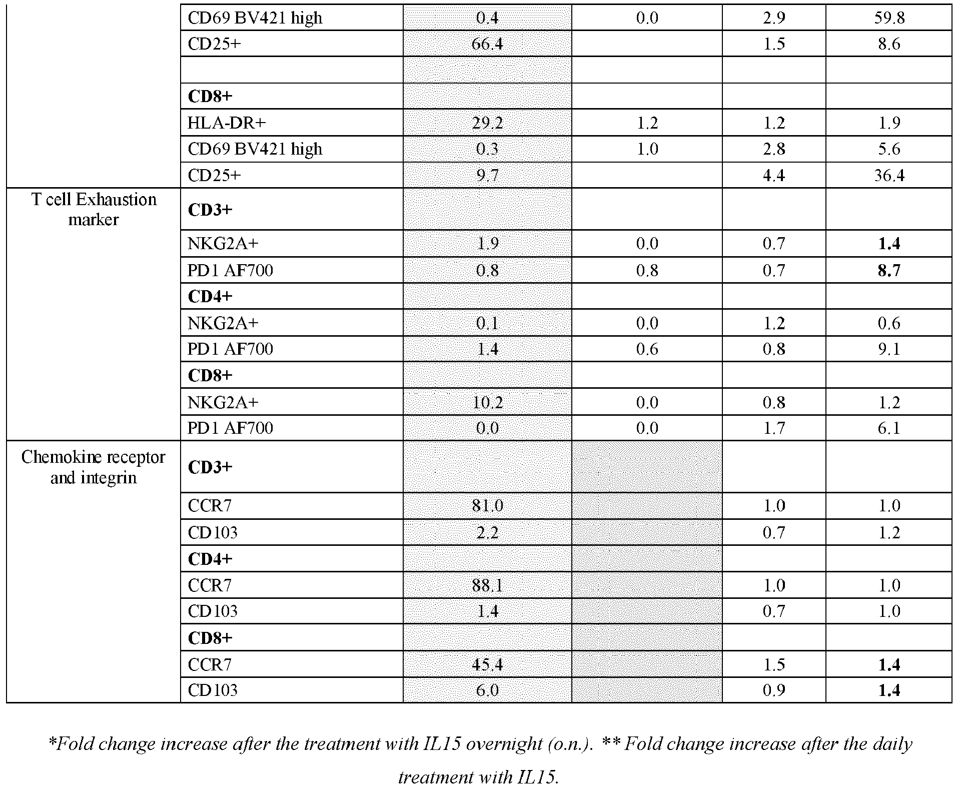

Most of the cells within the CD45RA- population were CD4+ as expected. 83.6% of the cells were CD45RA-CD3+CD4+ and 14.4% were CD45RA-CD3+CD8+. No differences in the expression of IFN-γ was observed within these two subpopulations (0.4% vs 0.3%) when incubated with the three peptides.

Within the CM and EM compartments most of the CD45RA-CD3+CD4+ cells were CM (89.1% vs 10.9%) as in the CD45RA-CD3+CD8+ population (CM: 61.6% vs 38.3%). Both subpopulation CM and EM expressed IFN-γ after coculture with the three peptides.

Then, we wanted to characterise in-depth the phenotypic expression of activation, exhaustion and Treg markers within the CD45RA- population of the convalescent donor in the presence of the three peptides. We found that the CD45RA-CD3+ expressed the activation marker HLA-DR (19.4%) in both subsets of cells CD4+ (16.7%) and CD8+ (29.2%) and these cells expressed IFN-γ. The expression of the exhaustion marker NKG2A was higher within the CD8+ cells **(Table 3)**.

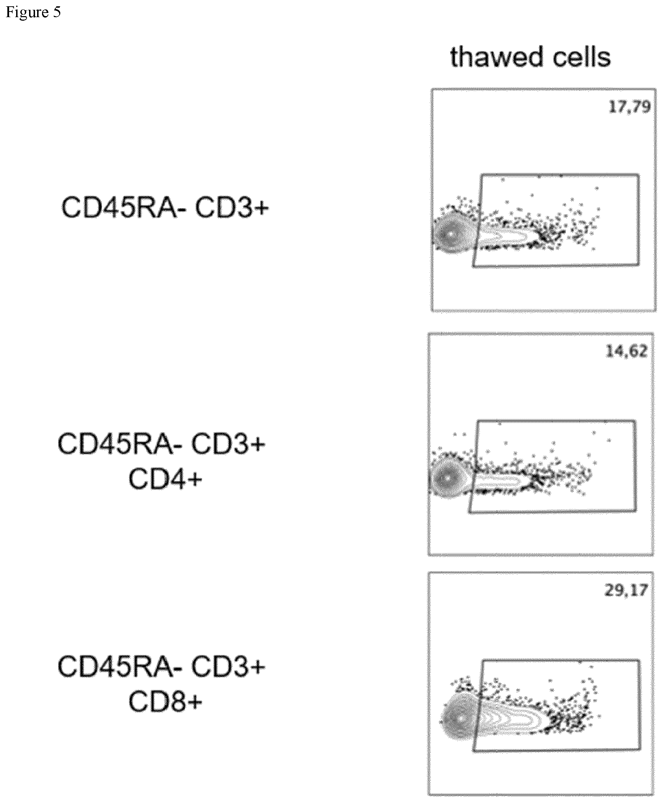

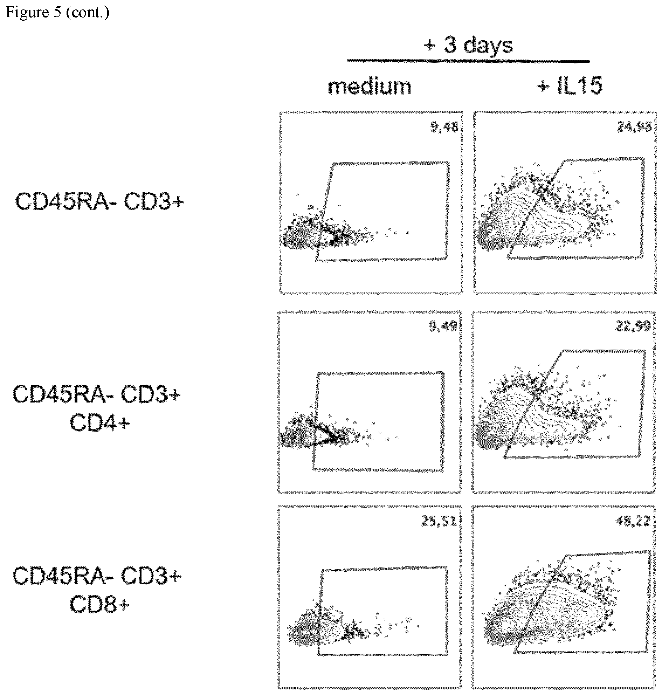

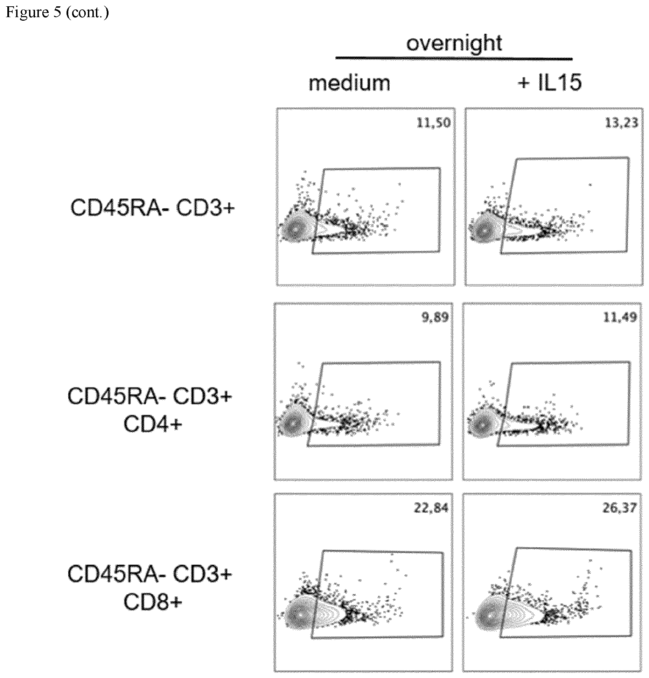

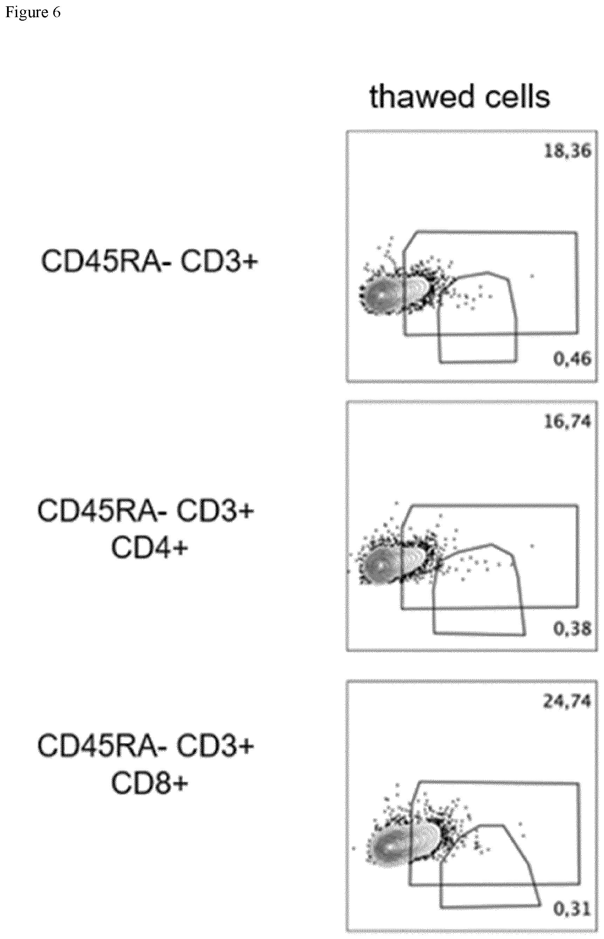

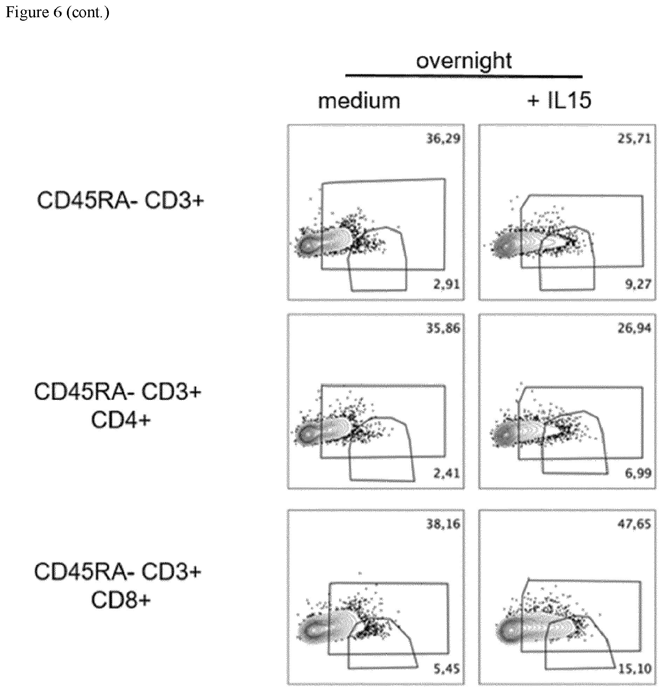

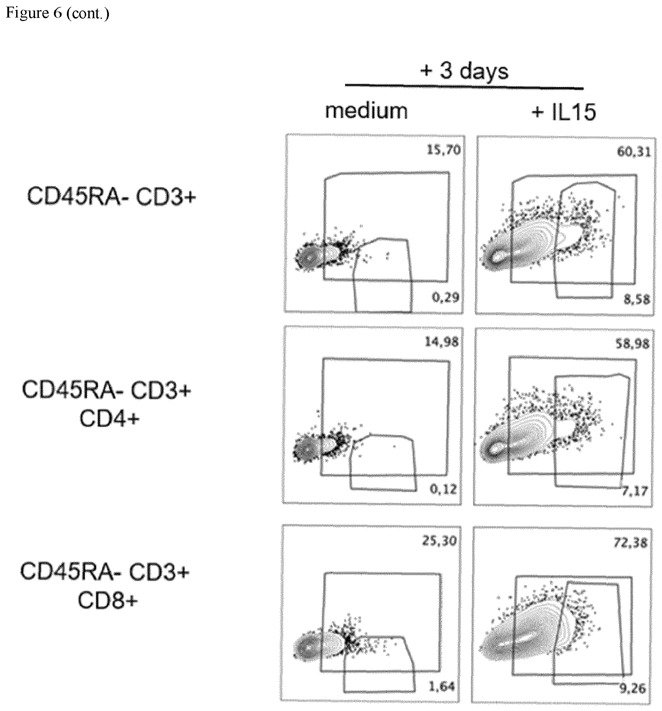

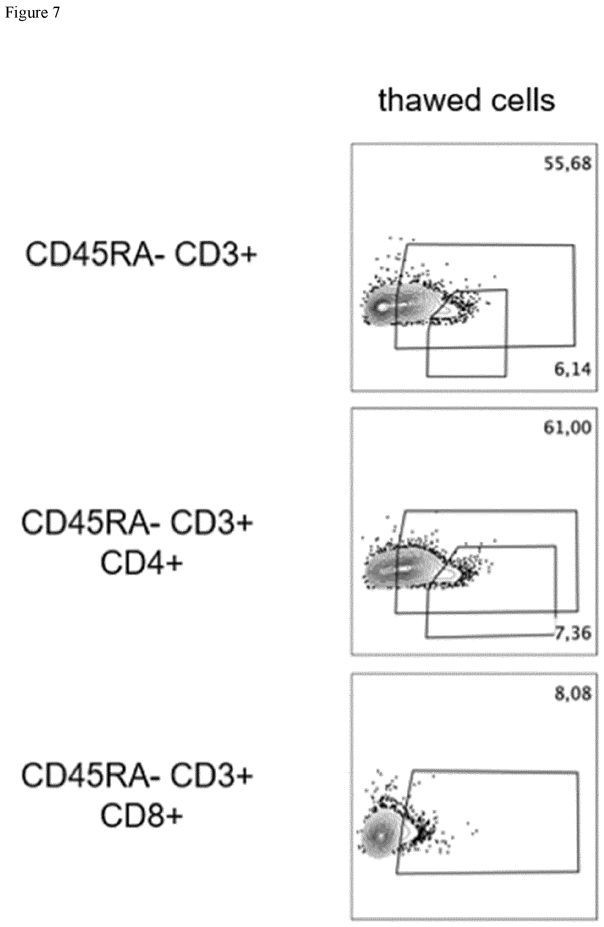

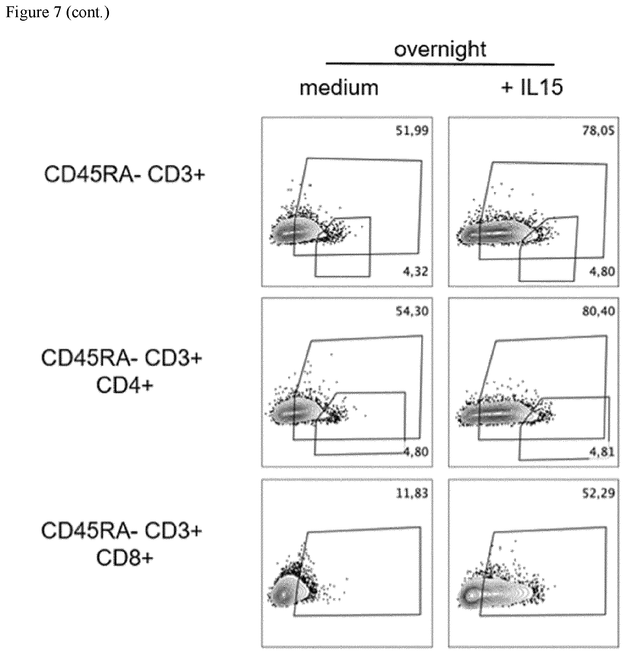

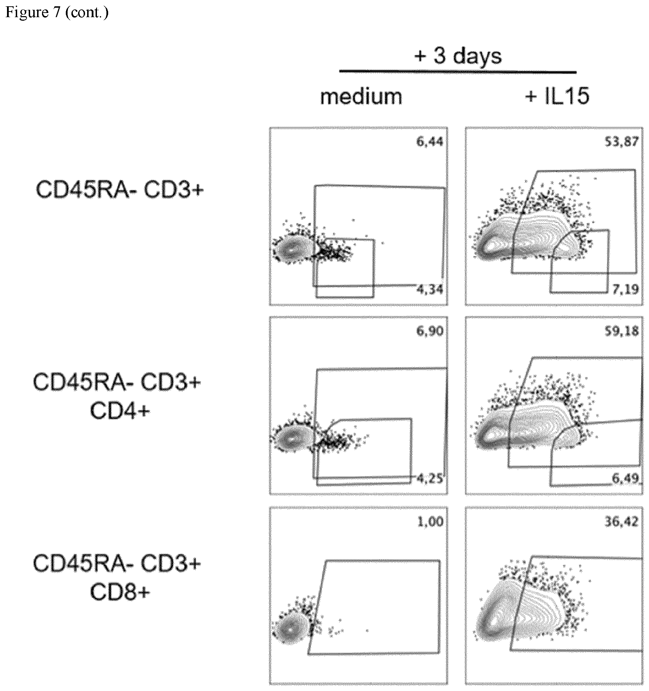

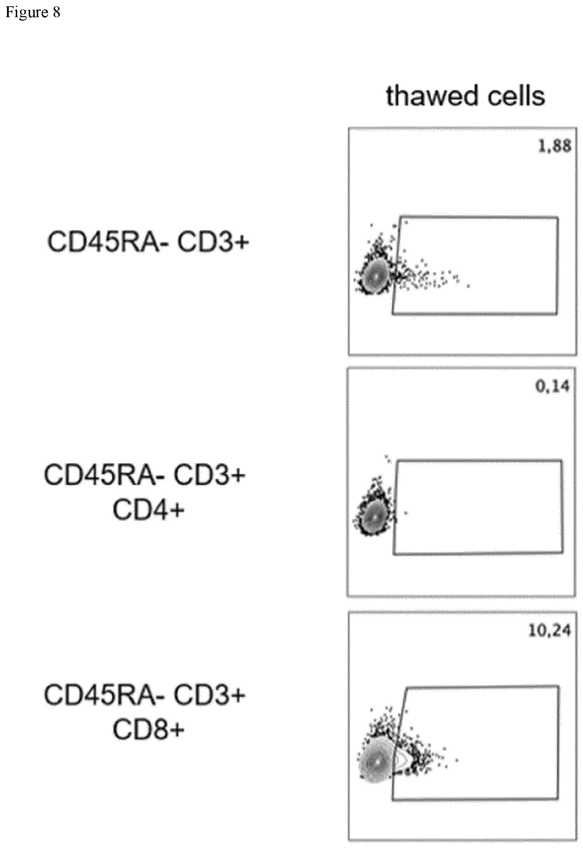

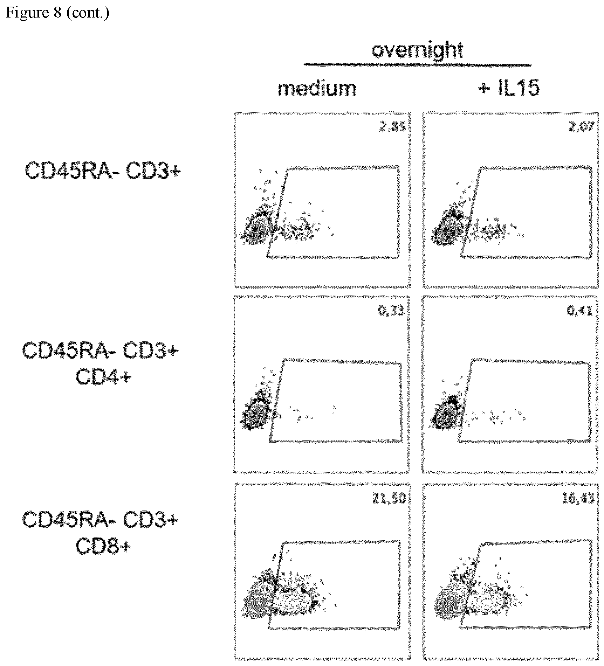

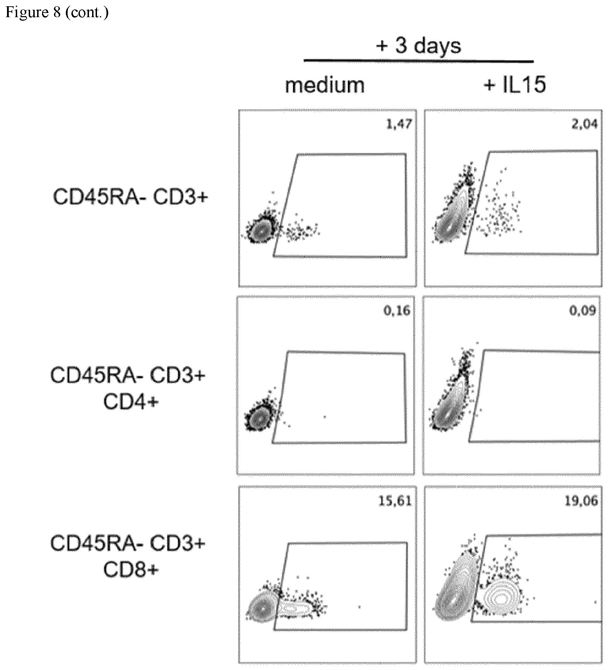

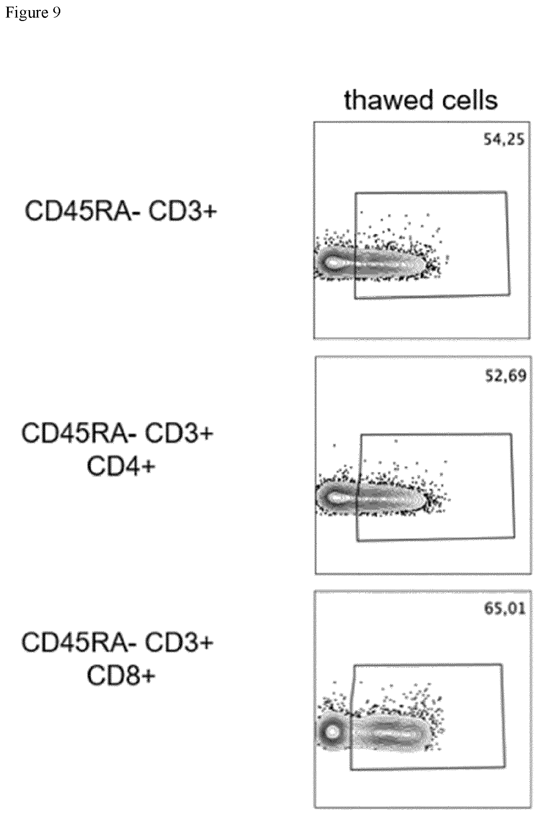

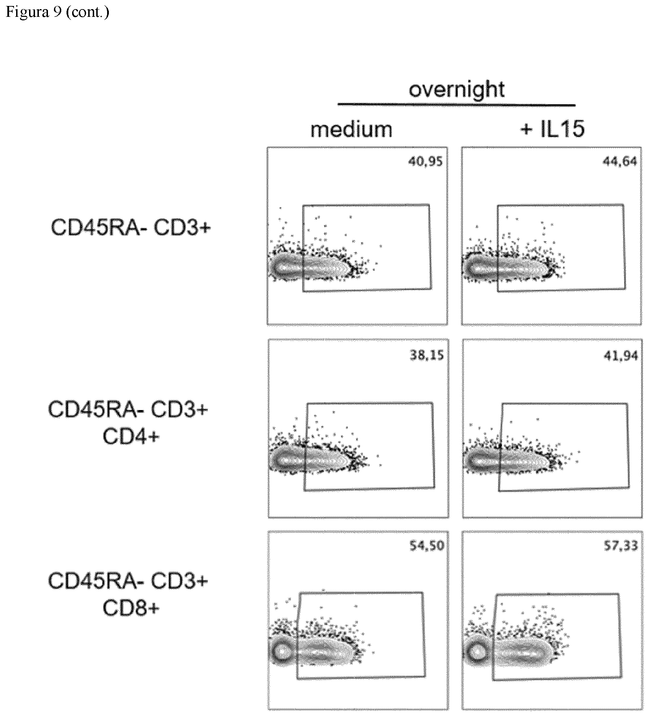

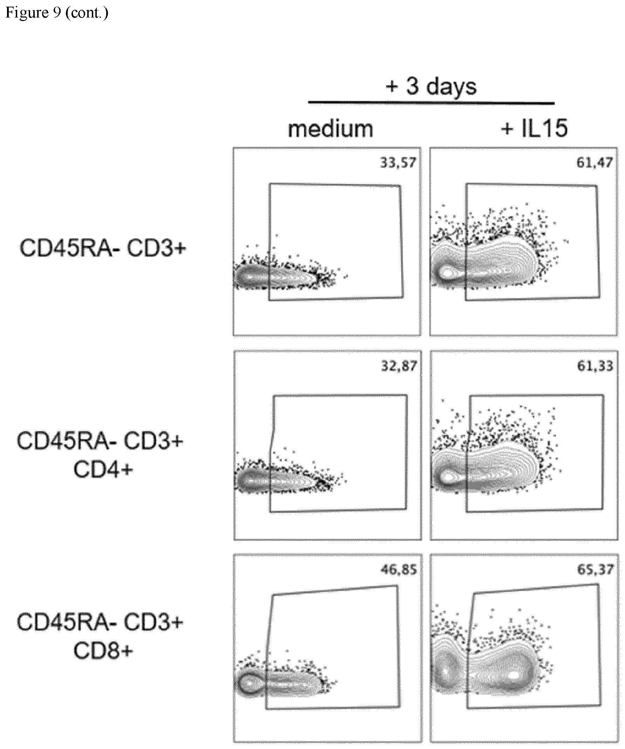

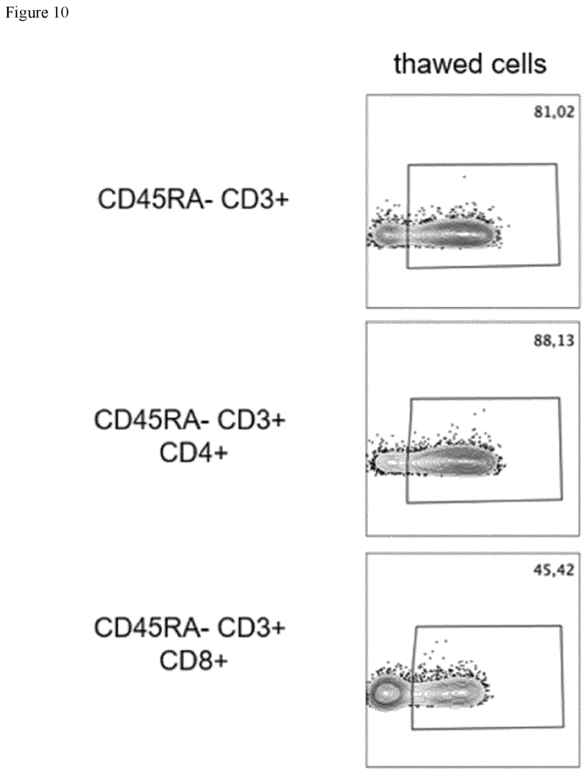

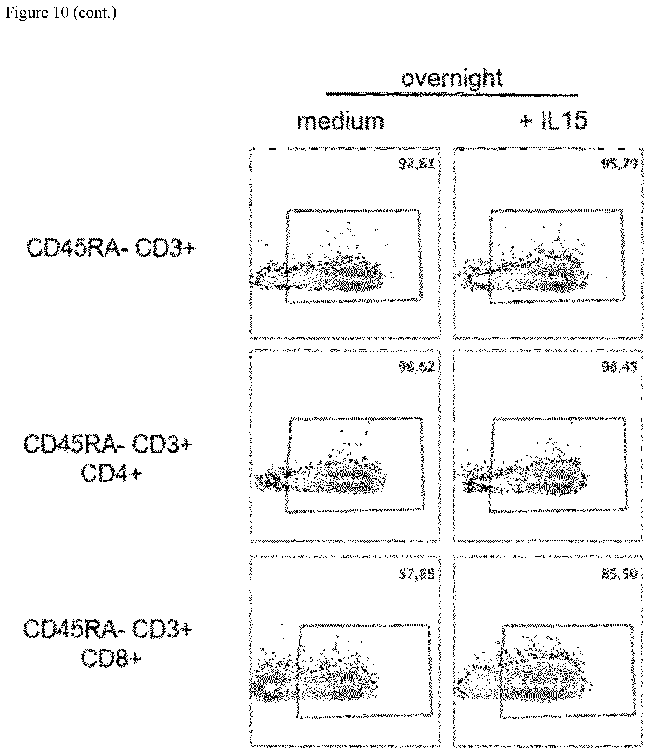

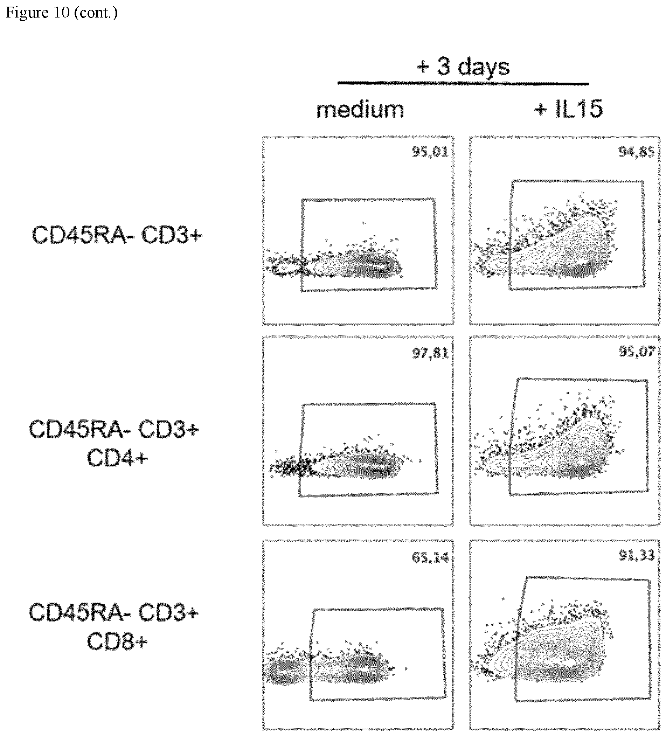

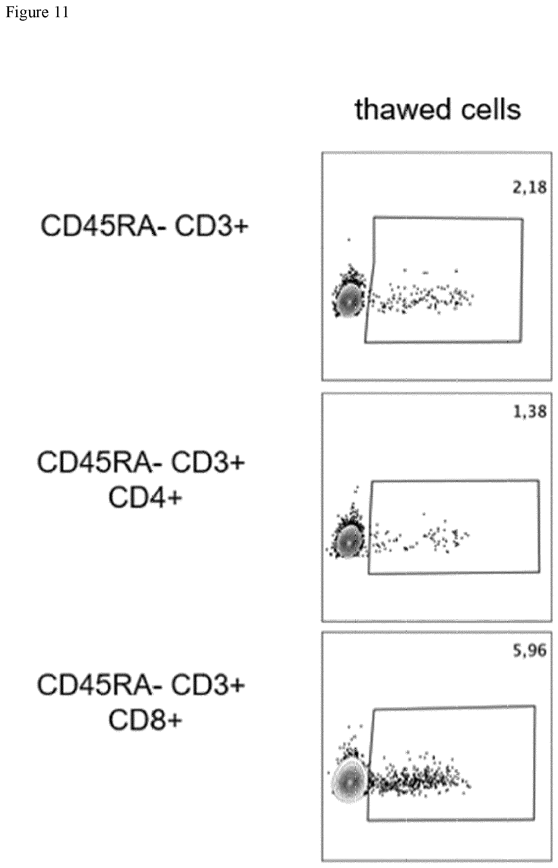

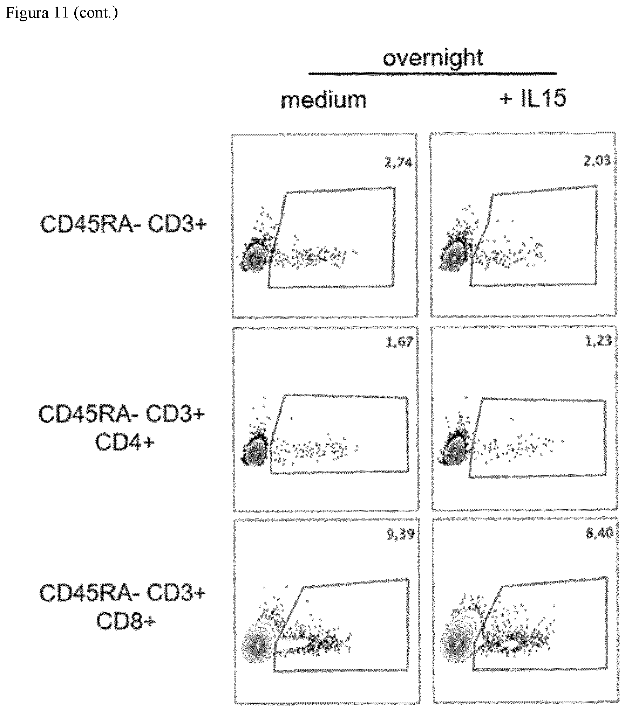

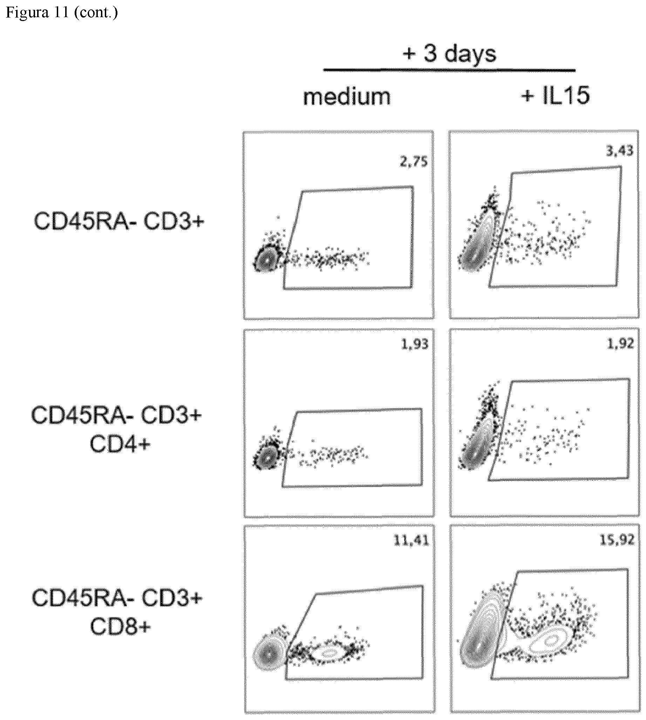

To induce a phenotype of memory T cells promoting survival and activation we incubated the CD45RA- T cells with IL15 o/n and for 72 hours a cytokine fundamental for memory T cells that induces the activation, the proliferation and the survival of T cells **(Table 3).** We observed a fold increase of the activation markers HLA-DR, CD69 and CD25 after 72 h incubation (2.6, 29.6, 8.5) than o/n incubation (1.2, 3.2, 1.5) when compared to a control with no cytokine. The expression of the exhaustion markers NKG2A and PD-1 were also higher after 72 hours incubation **(****Figures 5-9****).** Then, we look at the expression of cytokines associated to homing of T cells (CCR7 and CD103). There was a high expression in CD3+ cells of CCR7 (81%) being higher within the CD4+ subpopulation (88.1%) when compared to CD8+ cells (45.4%). The expression of CD103 was higher within the CD8+ when compared to CD4+ cells (6% vs 1.4%). When we look at the fold increase of these cytokines, we observed that although the fold increase was not very remarkable in the CD45RA-CD3+ cells after 72 hours incubation (1-fold CCR7 and 1.2-fold CD103), but this increase was higher (1.4) within the CD8+ subpopulation **(Table 3,** **Figure 10** **and** **11****).**

### Example 2.5. Large Clinical Scale production of CD45RA- memory T cells from a COVID-19 convalescent donor.

Leukapheresis was performed and CD45RA+ cell depletion was carried out in a CliniMACS Plus system.

60% of the cells were CD3+ and 99.7% cells were CD3+RO+. Most of the CD45RO+ cells were CD4+ with a high CD4/CD8 ratio **(Table 3).**

The design of a Phase I/II dose-escalation clinical trial to evaluate the safety of infusion of memory T cells for a 100 kg weight patient 3 cohorts will be planned with the following dose levels: first of up to 1x10⁵ /kg, second cohort 1x10⁵ to 5x10⁵ /kg and the third cohort 5x103 to 1x10⁶ kg. We calculate that the total number of CD3+ CD45RO+ cells expressing IFN-γ will be 1.56x10³ in the first cohort, 7.8x10³ in the second cohort and 1.6x10⁴ in the third cohort.

### Example 2.6 Infusion of memory T cells on COVID-19 patients

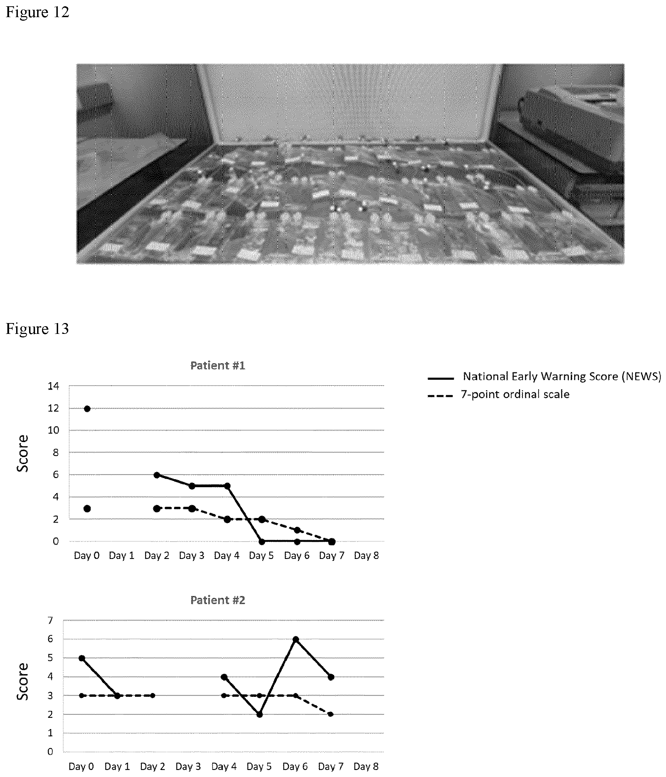

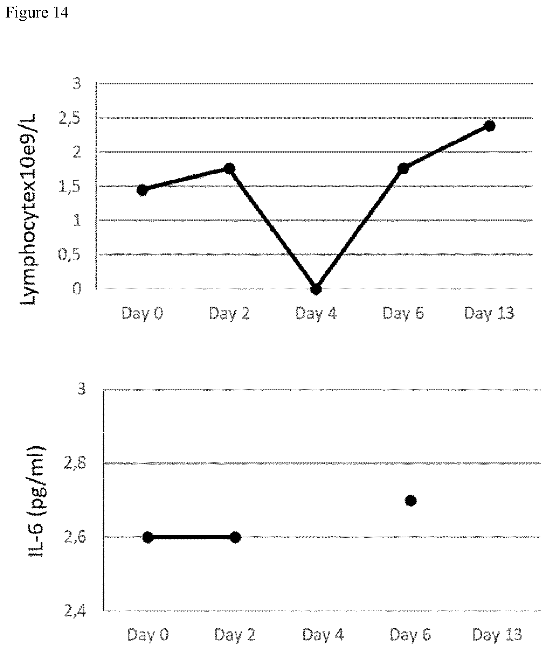

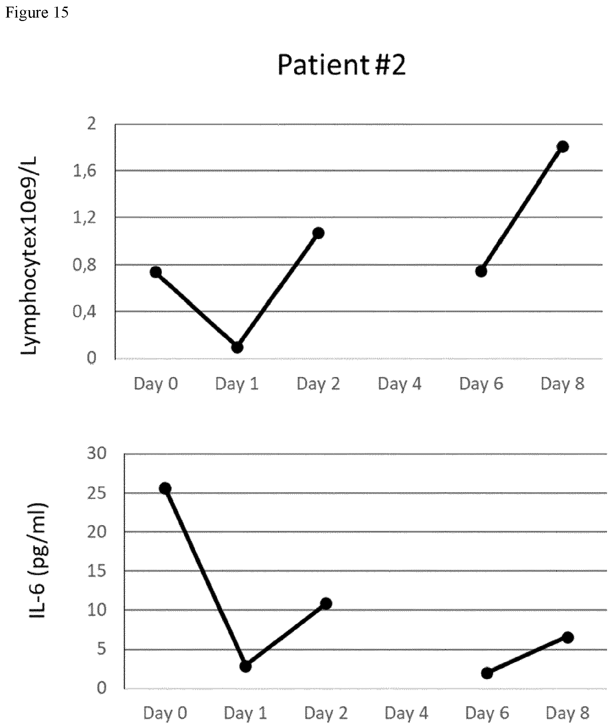

To date, 2 COVID-19 adult patients have been enrolled in phase I clinical trial which has been previously approved by the Ethical Committee of the La Paz University Hospital. The patients were hospitalized as suffering from COVID-19 pneumonia and lymphopenia, requiring oxygen. According with a cohort of patients treated with the standard of care at University Hospital La Paz, the patients enrolled in this study had some risk factors associated with bad outcome: age, male gender (patient #2), low oxygen saturation, low lymphocyte count and low C-reactive Protein. They were infused with a low dose (as compared with the usual dosage regime employed in the context of cell therapy) of up to 1x10⁵ /kg memory CD45RA- T cells, preferably 1x10⁵ /kg. The patients didn't show any signs of toxicity associate to the treatment. Both patients had good clinical response and recovered 6-7 days after infusion **(Table 4)**. The clinical parameters showed a quick a recovery too. Both patients decreased the scores reporting severity from day 0 to the time they were discharged **(****Figure 13****).** Lymphocyte counts increased also in both patients from day cero to the time they were discharged and the levels of IL-6 were in a normal range **(****Figure 14** and **Figure 15****).**

**Table 4**

| **Parameters** | ***La Paz cohort (2226)** | **Patient #1** | **Patient #2** |
|---|---|---|---|
| **Age (median, IQR)** | 61 (46-78) | 59 | 66 |
| **Male/Female (%)** | 48/52 | Female | Male |
| **Comorbidities (at least one), %** | 78.5 | No | Chronic heart disease |
| **Fever (%)** | 71.2 | Yes | Yes |
| **Cough (%)** | 61.7 | Yes | Yes |
| **Dyspnea** | 49.8 | Yes | Yes |
| **Median SatO2, % (IQR)** | 95 (92-97) | 89 | 91 |
| **Median time from disease onset to hospital admission, days (IQR)** | 6 (3-9) | 8 | 7 |
| **Complications during hospitalization, %** | 29 | No | No |
| **Lymphocyte count x10e9/L** | 0.93 (0.91-0.97) | 1.46 | 0.74 |
| **ALT U/L** | 31 (30-33) | 38 | 113 |
| **D-dimer, ng/mL** | 720 (680-758) | 1590 | 320 |
| **Serum ferritin, ng/L** | 346 (312-393) | 1563 | 705 |
| **IL-6, pg/mL** | 28.4 (18.8-40.1) | 2.6 | 25.6 |
| **LDH, U/L** | 321 (315-330) | 361 | 328 |
| **C-reactive protein, mg/L** | 71.1 (65.6-77.4) | 68.9 | 67.7 |
| **Recovery (days)** | | 6 | 8 |
| **HLA sharing** | | DQB1*02 | HLA-A*02:01; A*24:02 |
| **Pneumonia** | | Yes | Yes |
| **Days on supplemental oxygen** | | 4 | 6 |
| **Treatment Standard of Care (SoC)** | | Remdisivir, dexa | Remdisivir, dexa |
| **Treatment CD45RA-memory T cells (days after SoC)** | | 2 | 0 |
| **Lymphocyte count x10e9/L after 48 hours memory cell infusion** | | 1.76 | 1.07 |
| **Time of hospitalization since infusion (days)** | | 6 | 7 |

| | | | |
|---|---|---|---|
| ***Borobia AM, Carcas AJ, Arnalich F, et al. A Cohort of Patients with COVID-19 in a Major Teaching Hospital in Europe. J Clin Med. 2020;9(6):1733. Published 2020 Jun 4. doi:10.3390/jcm9061733 | | | |

## Claims

1. Cell suspension comprising at least 90% of CD45RA- memory T cells, **characterized in that** the memory T cells are derived from blood of convalescent patients recovered from an infection with Coronavirus and have specific lymphocyte antiviral reactivity against Coronavirus antigens, for use in the treatment of immunocompromised patients suffering from lymphopenia.

2. Cell suspension for use, according to claim 1, **characterized in that** the memory T cells are derived from blood of convalescent patients recovered from an infection with SARS-CoV-2 and have specific lymphocyte antiviral reactivity against SARS-CoV-2 antigens.

3. Cell suspension for use, according to any of the previous claims, wherein at least 75% of the CD45RA- cells are CD3+ cells, wherein at least 70% of the CD45RA- CD3+ cells are CD4+ and wherein at least 10% of the CD45RA- CD3+ cells are CD8+.

4. Cell suspension for use, according to any of the previous claims, wherein at least 60% of the CD45RA- CD4+ cells are CD27+, wherein at least 5% of the CD45RA- CD4+ cells are CD27- and wherein at least 5% of the CD45RA- CD4+ cells are CD25+.

5. Cell suspension for use, according to any of the previous claims, wherein at least 50% of the CD45RA- CD8+ cells are CD27+ and wherein at least 10% of the CD45RA- CD8+ cells are CD27-.

6. Cell suspension for use, according to any of the previous claims, wherein at least 10% of the CD3+ cells are HLADR+, wherein at least 0.5% of the CD3+ cells are CD69^{high+} and wherein at least 10% of the CD3+ cells are CD25+.

7. Cell suspension for use, according to any of the previous claims, wherein at least 5% of the CD4+ cells are HLADR+, wherein at least 0.2% of the CD4+ cells are CD69+, wherein at least 20% of the CD4+ cells are CD25+.

8. Cell suspension for use, according to any of the previous claims, wherein at least 5% of the CD8+ cells are HLADR+, wherein at least 0.15% of the CD8^{high+} cells are CD69+, and wherein at least 4% of the CD8+ cells are CD25+.

9. Cell suspension for use, according to any of the previous claims, wherein less than 5% of the CD3+ cell are NKG2A+, wherein less than 1 % of the CD3+ cells are PD1+, wherein less than 0.1% of the CD4+ cells are NKG2A+, wherein less than 5% of the CD4+ cells are PD1+, wherein less than 20% of the CD8+ cells are NKG2A+ and wherein less than 5% of the CD8+ cells are PD1+.

10. Cell suspension for use, according to any of the previous claims, wherein at least 60% of the CD3+ cells are CCR7+, wherein at least 1% of the CD3+ cells are CD103+, wherein at least 50% of the CD4+ cells are CCR7+, wherein at least 0.5% of the CD4+ cells are CD103+, wherein at least 30% of the CD8+ cells are CCR7+ and wherein at least 2% of the CD8+ cells are CD103+.

11. Cell suspension for use, according to any of the previous claims, wherein the expression of the activation markers CD69, CD25, HLADR and/or CD103 is **characterized by** a fold change of at least 1.5 when compared with the expression measured in the basal cell population and consequently show an improvement in activation markers and migration capacity to the respiratory track.

12. Cell suspension for use, according to any of the previous claims, as adoptive third-party off-the-shelf treatment in patients suffering from lymphopenia caused by a viral infection, preferably caused by Coronavirus.

13. Cell suspension for use, according to any of the previous claims, in the treatment of immunocompromised patients suffering from lymphopenia caused by a viral infection, preferably caused by SARS-CoV-2.

14. Cell suspension for use, according to any of the previous claims, wherein the cell suspension is administered either intravenously, by nebulization or locally in the oral, nasal or ocular mucosae.

15. Pharmaceutical composition comprising the cell suspension of any of the claims 3 to 14 and, optionally, pharmaceutically acceptable excipients and/or carriers.
